# EUROPÄISCHE PATENTANMELDUNG

(11) **EP 1 216 706 A2**
(43) Veröffentlichungstag der Anmeldung: **26.06.2002**
(21) Anmeldenummer: 01130635.4
(22) Anmeldetag: 21.12.2001
(51) Int. Cl.: A61K 35/74, A61K 47/38, A61P 37/04

(54) **Enterale Zubereitung zur Stärkung des Immunsystems**

(30) Priorität: 22.12.2000 DE 10064732
(71) Anmelder: Maxim Markenprodukte GmbH & Co. KG, 50259 Pulheim-Brauweiler (DE)
(72) Erfinder: Ko, Hong Lioe, DR., 50968 Köln (DE); Pulverer, Gerhard, 50858 Köln (DE); Beuth, Josef, 50931 Köln (DE); Giesen, Rolf, 50858 Köln (DE)
(74) Vertreter: Meyers, Hans-Wilhelm, Dr.Dipl.-Chem.

(57) **Zusammenfassung**

Enterale Zubereitung mit mindestens einer Propionibakterienart, ausgenommen Propioni bacterium acnes, und ein matrixbildendes Mittel.

## Beschreibung

Gegenstand der vorliegenden Erfindung ist eine enterale Zubereitung sowie die Verwendung der erfindungsgemäßen enteralen Zubereitung.

Das körpereigene Abwehrsystem (Immunsystem) beeinflußt insbesondere den Verlauf von Infektionskrankheiten (u.a. grippale Infekte, Bakterien-, Pilzund Parasitenbefall), Tumorerkrankungen, sowie allergische Reaktionen (u.a. Neurodermitis). Daher ist die Stärkung dieses Organsystems eine wesentliche Voraussetzung, derartige Erkrankungen in Entstehung und Ausbreitung zu hemmen.

Bakterien und Bakterienprodukte sind seit dem Altertum als Aktivatoren des Abwehrsystems bekannt. Bereits vor der antibiotischen/chemotherapeutischen Zeit wurden bakterielle Immunstimulantien zur Behandlung von Infektionskrankheiten und zur Tumortherapie mit Erfolg eingesetzt. Diese Therapieform gewinnt mit zunehmender Resistenzentwicklung von Bakterien und Tumorzellen und damit einhergehendem Therapieversagen derzeit an Gewicht.

Zur Vorbeugung und Therapie bösartiger, infektiologischer und allergischer Erkrankungen eröffnen sie ein neues Spektrum

Das der Erfindung zu Grunde liegende technische Problem besteht mithin in der Bereitstellung eines Systems mit dem das Immunsystem gestärkt werden kann.

Gelöst wird dieses Problem durch eine enterale Zubereitung mit mindestens einer Propionibakterienart ausgenommen Propioni bacterium acnes und ein matrixbildendes Mittel.

Vorzugsweise ist bei der erfindungsgemäßen enteralen Zubereitung das matrixbildende Mittel ausgewählt aus der Gruppe bestehend aus mikrokristalliner Cellulose.

Insbesondere werden die folgenden Propionibakterien eingesetzt: Propionibacterium avidum KP-40.

Vorzugsweise liegen die Propionibakterien in einer Menge von 1x10⁵ bis 1x10⁶ abgetöteter Bakterien pro Einheit in der enteralen Zubereitung gemäß der Erfindung vor.

Die erfindungsgemäßen enteralen Zubereitungen liegen vorzugsweise in Form von Tabletten, Suppositorien, Pulvern, Kapseln und Lutschtabletten vor.

Erfindungsgemäß wird die Verwendung von mindestens einer Propionibakterienart, ausgenommen Propioni bacterium acnes, zur Herstellung einer enteralen Zubereitung als Arzneimittel zur Behandlung von mit einem geschwächten Immunsystem in Zusammenhang stehenden Erkrankungen sowie als Nahrungsergänzungsmittel zur Stärkung des Immunsystems bei stressbedingten, jahreszeitlichen oder anderer Schwächung sowie infektiologischen, malignen, autoimmunen Erkrankungen beansprucht.

Die mit dem geschwächten Immunsystem in Zusammenhang stehenden Erkrankungen sind insbesondere aus der Gruppe bestehend aus banalen saisonalen Infektionen, allergischen Erkrankungen, infektiösen Erkrankungen, malignen Erkrankungen, Autoimmun-Erkrankungen ausgewählt.

Propionibakterien (P. avidum KP-40) gehören zur normalen Hautflora des Menschen und haben sich in experimentellen und klinischen Studien als äußerst wirksame Immunstimulantien erwiesen.

Mit einer wissenschaftlich ausgetesteten probiotischen Nahrungsergänzung kann hierzu ein sinnvoller Beitrag geleistet werden.

Die Erfindung wird am folgenden Ausführungsbeispiel näher erläutert.
Nahrungsergänzung
Inaktivierte Propionibacterium avidum KP-40 1x10⁵ bis 1x10⁶ /Einheit
Inaktivierte (abgetötete) Propionibacterium avidum KP-40, mikrokristalline Cellulose
in Kapsel enthält 1x10⁵ bis 1x10⁶ abgetöteter, lyophilisierter P.avidum KP-40
Gefriergetrocknete Bakterien (Lyophilisat) aus einer Aufschwemmung (Suspension) von 1x10⁵ bis 1x10⁶/mL Propionibacterium avidum KP-40.
Mikrokristalline Cellulose.

## Patentansprüche

1. Enterale Zubereitung mit mindestens einer Propionibakterienart ausgenommen Propioni bacterium acnes und ein matrixbildendes Mittel.

2. Enterale Zubereitung nach Anspruch 1, wobei das matrixbildende Mittel mikrokristalline Cellulose ist.

3. Enterale Zubereitung nach Anspruch 1 und/oder 2, wobei Propionibacterium avidium KP-40 eingesetzt wird.

4. Enterale Zubereitung mindestens nach einem der Ansprüche 1 bis 3, wobei die Propionibakterien in einer Menge von 1x10⁵ bis 1x10⁶ pro Einheit vorliegen.

5. Enterale Zubereitungen nach mindestens einem der Ansprüche 1 bis 4, wobei die Zubereitungen in Form von Tabletten, Suppositorien, Pulvern, Kapseln und Lutschtabletten vorliegen.

6. Verwendung von mindestens einer Propionibakterienart, ausgenommen Propionibacterium acnes, zur Herstellung einer enteralen Zubereitung als Arzneimittel zur Behandlung von mit einem geschwächten Immunsystem in Zusammenhang stehenden Erkrankungen sowie als Nahrungsergänzungsmittel zur Stärkung des Immunsystems. bei stressbedingten, jahreszeitlichen bzw. anderer Schwächung sowie infektiologischen, malignen, autoimmunogen Erkrankungen.

7. Verwendung nach Anspruch 6, wobei die mit dem geschwächten Immunsystem in Zusammenhang stehenden Erkrankungen ausgewählt sind aus der Gruppe bestehend aus banalen saisonalen Infektionen, allergischen Erkrankungen, infektiösen Erkrankungen, malignen, autoimmunen Erkrankungen.
